# EUROPEAN PATENT SPECIFICATION

(11) **EP 0 956 318 B1**
(45) Date of publication and mention of the grant of the patent: **11.01.2006**
(21) Application number: 98900771.1
(22) Date of filing: 20.01.1998
(51) Int. Cl.: C09D 4/00, C08F 220/36, C07C 233/00

(54) **RADIATION-CURABLE COMPOSITION**
STRAHLUNGSHÄRTBARE ZUSAMMENSETZUNG
COMPOSITION POUVANT DURCIR PAR RAYONNEMENT

(30) Priority: 30.01.1997 NL 1005129
(43) Date of publication of application: 17.11.1999
(73) Proprietor: DSM IP Assets B.V., 6411 TE Heerlen (NL)
(72) Inventor: MEIJ, Theodorus, Hendrik, NL-8011 VC Zwolle (NL); HOUWELING, Marten, NL-8011 HG Zwolle (NL); DIAS, Aylvin, Jorge, Angelo, Athanasiu, NL-6221 JD Maastricht (NL); JANSEN, Johan, Franz, Gradus, Antonius, NL-6165 AP Geleen (NL); VAN BENTHEM, Rudolfus, Antonius, Theodorus, Maria, NL-6141 BR Sittard (NL)
(86) International application number: PCT/NL1998/000035
(87) International publication number: WO 1998/033855

(56) References cited:
- EP-A- 0 263 749
- EP-A- 0 448 399
- EP-A- 0 499 923
- EP-A- 0 685 535
- US-A- 3 366 613

## Description

The invention relates to a radiation-curable composition.

During radiation curing processes the transformation of the fluid applied film to a solid crosslinked network can be considered to progress through three distinct stages being induction, polymerisation and attainment of maximum cure plateau. (Chemistry and Technology of UV and EB formulations, Volume IV, Oldring, 1991, pages 8-12).

Factors which improve or inhibit cure rate are, for example, the lamp system (UV-dose, intensity, wavelength, IR-content) and the chemical system (reactivity, absorption, coating weight, pigmentation, temperature, oxygen inhibition and substrate).

For commercial coating operations, it is necessary that the coating achieves a tackfree surface within seconds or less, because the Interval between application of the coating and stacking or rewinding of the coated substrate is very short. Failure of the coating to achieve a non-tacky surface in this brief interval will result in the layers of coated substrate (in a stack or roll) sticking together ("blocking).

It is the object of the present invention to provide a coating composition having a high cure rate or rate of polymerisation and having the desired chemical and mechanical properies.

The UV or EB curable coating composition according to the invention comprisies a mono or multivalent carboxylic ester of a β-hydroxyalkylamide group containing compound and a photoinitiator, in which the carboxylic ester is derived from an α,β-ethylenically unsaturated carboxylic acid wherein the compound is a compound according to formula (I): 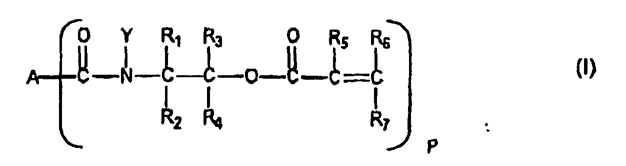
where:
A = a monovalent or polyvalent organic group which is derived from a saturated or an unsaturated (C₁-C₆₀) alkyl group, or derived from an (C₆-C₁₀) aryl group,
Y = hydrogen, a (C₁-C₈) alkyl group or 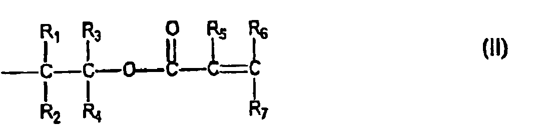
R₁, R₂, R₃, R₄ are identical or different, hydrogen or a linear, branched or cyclic (C₁-C₈) alkyl chain,
R₅ = hydrogen, (C₁-C₅) alkyl, -CH₂OH or CH₂COOX,
R₈, R₇ = hydrogen, (C₁-C₈) alkyl, (C₆-C₁₀) aryl or COOX
X = hydrogen or (C₁-C₈) alkyl and
p = 1 or 2 and
wherein the photoinitiator has ketone functionality, the photoinitiator is an acyl phosphine or the photoinitiator is of the Norrish-II-type.

The composition according to the invention results in high maximum polymerization rates.

R¹, R² or R³ may form part of a cycloalkyl group.

The organic groups in A may be substituted with, for example, ethers, esters, hydroxyl, amides, acids, amines or ketones.

Preferably, ester- or hydroxylgroups are applied as substitutents.

Preferably, A is a monovalent organic group which is derived from a saturated (C₁-C₁₂) alkyl group.

According to another preferred embodiment of the invention A is a polyvalent organic group derived from a saturated (C₂-C₁₀) alkyl group or a C₆-aryl group.

Preferably, Y is hydrogen or methyl.

Preferably, R¹, R², R³ and R⁴ are hydrogen or methyl.

R⁵ is preferably hydrogen or (m)ethyl.

R⁶ and R⁷ are preferably hydrogen.

The compound applied in the invention generally has a number-average molecular weight (Mn) of between 140 and 2500, and preferably of between 450 and 1000.

The compound can be obtained, for instance, by an esterification reaction between a β-hydroxyatkytamide and an unsaturated carboxylic acid, at a temperature between, for example, 80° C and 140° C.

Preferably, 1 - 1.5 mol of acid are used per mole of hydroxide.

Preferably, the reaction takes place in the presence of an organic solvent, such as, for example, xylene, toluene or tetrahydrofuran.

Preferably, the reaction takes place in the presence of a stabilizing compound which prevents polymerization of the unsaturated ester groups under the conditions used for effecting this reaction. The stabilising compound or a mixture of stabilising compounds is generally used in amounts between about 50 and about 2000 ppm and preferably between 75 and 1000 ppm. They can be used in aerobic or anaerobic conditions depending on the stabilising compound.

Suitable stabilizing compounds include, for example, hydroquinone, monomethylhydroquinone, anthraquinone, β-nitrostyrene, phenothiazine and 2,6-di-tert-butyl-4-methyl-phenol (BHT).

The esterification reaction may take place in the presence of a catalyst. Suitable catalysts include strong acids, for example, sulphur-containing organic acids like alkane sulphonic acids and methane sulphonic acid.

Suitable unsaturated carboxylic acids include, for example, (meth)acrylic acid and derivatives, crotonic acid, (semi-ester of) itaconic acid, maleic acid, citaconic acid, mesaconic acid and fumaric acid.

Suitable β-hydroxyalkylamides include, for example, N,N'-bis(di-β-hydroxyethyl)-1,6-hexanediamide, N-di-β-hydroxyethyl acetamide, N,N-bis(di-p-hydroxypropyl)-1,6-hexanediamide, N-di-β-hydroxypropyl acetamide, N-di-β-hydroxyethyl benzamide and N-di-β-hydroxypropyl benzamide. The compound applied in the invention can also be obtained by the reaction between of a β-hydroxyalkyl amide and an unsaturated carboxylic acid chloride, anhydride or ester.

The reaction between the amide and the unsaturated chloride or anhydride preferably takes place at temperature between 0° C and 30° C in a solvent in the presence of a base. Suitable solvents include, for example, tetrahydroferan, water, dichloromethane or diethylether. Suitable bases include, for example, pyridine or triethylamine.

Suitable chlorides, anhydrides or esters include the chlorides, anhydrides and esters of the in the foregoing mentioned carboxylic acid.

The reaction between the amide and the unsaturated ester, preferably, takes place at temperatures between 80° C and 140° C in the presence of a Lewis acid.

Preferably, an excess of the unsaturated ester is applied. The ester functions both as solvent and as reactant.

Suitable Lewis acids are, for example, tetra alkyl titanate and sulphuric acid.

Another process for the preparation of the compound applied in the invention is the reaction between an oxazoline and an unsaturated carboxylic acid.

Such a reaction can, for example, take place between 50° C and 140° C.

Suitable oxazolines include, for instance, oxazoline and (C₁-C₂₀) alkyloxazolines, for instance, ethyl oxazoline and undecyloxazoline.

Suitable unsaturated carboxylic acids include, for example, (meth)acrylic acid and derivatives, crotonic acid, (semi-ester of) itaconic acid, maleic acid, citaconic acid, mesaconic acid and fumaric acid.

Preferably, methauyllc acid and acrylic acid are used.

The compound applied in the invention can be cured by means of a free-radical reaction. In these reactions the free radicals can be obtained by radiation initiation.

Radiation-curing preferably takes place by means of, for example, a photochemical process such as, for example, ultraviolet radiation (UV) or a radiation-chemical process such as electron beam (EB).

UV and EB radiation are explained in greater detail by for example Bett et al. in the article entitled "UV and EB curing" (Jocca 1990 (11), pages 446 - 453).

The amount of the compound according to formule (I) can range between 0,01% by weight and 100% by weight in the composition according to the invention.

Generally, the radiation curable composition according to the invention is substantiatly solvent free.

The composition according to the invention can be used, for example, in coating compositions, inks and adhesives.

If desired and depending on the application, the compound can be combined with oligomers or polymers which are based, for example, on (meth)acrylate units, maleate units, fumarate units, itaconate units, vinylester units and/or vinylether units.

Due to the relatively high cure speeds the present compounds can also be applied as additives for enhancing the cure speed of a formulation. In general such additives are used in amounts ranging between 0,01% and 25% by weight and preferably in amounts between 0,5% and 10% by weight relatively to the total amount of all ingredients.

After curing the coatings according to the invention have many desired properties such as for example good chemical properties (resistance to solvents, acids, alkalis and moisture), good optical properties and appearance, good mechanical properties (such as hardness, flexibility, adhesion, abrasian resistance, strength and durability), good thermal stability and good weatherability.

The composition comprising the radiation curable binder composition may further comprise pigments, stabilisers and other additives.

The radiation curable formulation generally consists of a prepolymer, a reactive diluent and additives. Two other possible components, depending upon the type of formulation and cure mechanism are pigments and photoinitiator system.

The composition can be applied as a water based coating, as a solvent based coating, as a high solids coating and as a 100% solids coating.

According to a preferred embodiment of the invention the composition is applied as a powder coating.

The ester applied in the present invention can also be used as a crosslinker in powder coating compositions if the compound is composed in such a way that the softening point (glass transition temperature or melting point) is sufficiently high to be used in this application. Generally, this temperature has to be higher than 40° C.

The most preferred irradiation source is ultraviolet light. Ultraviolet light is preferably high intensity light to provide a dosage to achieve reasonable curing rates. In the event that lower energy light is to be applied, it may then be desired to subject the compositions also to elevated temperatures in order to reduce the time for adequate polymerization to occur.

With respect to UV curing equipment we refer to, for example, pages 161-234 of Chemistry and Technology of UV and EB-formulations, Volume 1, Oldring, 1991.

Suitable lamps employed to provide the desired high intensity and availability of wavelength and spectral distribution include for example that available from Fusion Systems. Corp.

A composition according to the present invention can be applied on substrates such as, for example, plastic, paper, board, leather, glass, wood and metal.

This composition is preferably polymerised in the presence of a photoinitiator but it is also possible to polymerise in the absence of a photoinitiator.

Suitable photoinitiators allow for initiation of the curing process with exposure to light having wavelengths between about 200 nm and about 600 nm. Suitable photoinitiators have ketone functionalities and can be aromatic such as, for example, benzophenone Darocur 1173® (Ciba) is a suitable benzyl-ketal-based photoinitiator, which contains 2-hydroxy-2-methyl-1-phenylpropane-1-one as an active component. lrgacure 184® (Ciba) is an aryl ketone containing hydroxycyclohexyl phenyl ketone as active component, and is a suitable photoinitiator. Irgacure 369® (active component 2-benzyl-2-dimethylaminol-1-(4-morpholinophenyl)-butanone-1) is also suitable. Acyl phosphines, such as for example 2,4,6,-trimethylbenzoyl diphenyl phosphone oxide (Lucerine TPO®, BASF) can also be used, as can Quantacure CPTX® (Octel Chemicals), which contains 1-chloro-4-propoxy thioxanthone as active component. Chemical derivatives of these photoinitiators are suitable, as are mixtures of these photoinitiators. A suitable combination of photoinitiators is lrgacure 1800™ (Ciba) consisting of 75% by weight lrgacure 184™ and 25% by weight (bis-(2,6-dimethoxy benzoyl)-2,4,4-trimethylpentylphosoxide). Other suitable photoinitiators can be of the Norrish-II-type, for example, the combinations benzophenone with amine, maleimide with amine, thioxantone with amine and antrachinon with amine.

EP-A-685535 relates to UV-curabte coating compositions curable at high speed. The acrylate compound differs from the acrylate compound as used in the coating composition of the present invention because the group which forms a (CH₂)₃₋ring between N and C=O is not present in the radiation curable composition according to the invention.

EP-A-499923 relates to substances for use as adhesive for fillings of teeth. Adhesives constitute a completely different field as coatings, having a different set of requirements to fulfill.

EP-A-3366613 and EP-A-448399 are directed to thermally cured coating compositions.

The invention is explained by reference to the following non-restrictive experiments and examples.

In the following the cure behaviour monitored with "real time infra red spectroscopy". The conversion of the double bonds during the photopolymerisation was monitored by means of infrared (Bruker IFS55).

### Experiment I

### Preparation of N,N-bis(β-hydroxyethyl)acetamide

315 g of diethanolamine were dissolved in 500 g of tetrahydrofuran In a round-bottom flask. Then, 310 g of acetic anhydride were slowly added. The temperature was kept below 5° C with the aid of a cooling bath. When, after the dropwise addition, exothermic heat was no longer detectable, heating was slowly carried out to 100° C. Tetrahydrofuran and acetic acid were removed under vacuum.

### Experiment II

### Preparation of N,N-bis(β-acryloxyethyl)acetamide

100 grams of the product obtained according to Experiment I, 300 grams of toluene, 0.05 gram of hydroquinone monomethyl ether and 147 grams of acrylic acid were combined in a round-bottom flask (1 litre). The solution was azeotropically distilled for 9 hours. After cooling to room temperature, the excess acrylic acid was neutralized with a saturated solution of sodium bicarbonate in water and the pH was adjusted to 12. After separation of the water layer and the toluene layer, the water layer was again extracted with toluene. The combined toluene layers were dried by distilling under vacuum.

### Experiment III

### Preparation of N,N',N,N'-tetrakis(β-acryloxyethyl)-1,6-hexanediamide

Experiment II was repeated with the exception that 320 grams of N,N'-bis(di-β-hydroxyethyl)-1,6-hexanediamide (Primid® XL 552; Rohm & Haas) and 432 grams acid of acrylic acid were mixed in order to obtain N,N',N,N'-tetrakis(β-acryloxyethyl)-1,6-hexanediamide.

### Example I

### Curing of a mixture comprising N,N-bis-(β-acryloxyethyl) acetamide

0.99 g of N,N-bis-(β-acryloxyethyl)acetamide according to Experiment II and 0.01 g of Irgacure 184™ were homogenously mixed at room temperature. This mixture was applied as a 10 µm thick film on a gold coated alumina plate.

Subsequently this plate with the film was irradiated in the infrared machine with a dose of 500mW/cm² and the conversion of the double bonds was monitored:
- maximum rate of polymerisation 46,0 mol kg⁻¹s⁻¹

For the calculations of the rates when molecules with functionalities higher than 1 are used, the molecular weight per acrylate unit is defined as the total molecular weight of the molecule divided by the number of acrylate functionalities.

### Example II

### Curing of a mixture comprising N,N',N,N'-tetrakis(acryloxyethyl)1,6-hexanediamide

0.99 g of the diamide according to Experiment III and 0.01 g of irgacure 184™ were homogenously mixed at room temperature. This mixture was applied as a 10 µm thick film on a gold coated alumina plate.

Subsequently this plate with the film was irradiated with a dose of 500mW/cm² and the conversion of the double in the infrared machine bonds was monitored:
- maximum rate of polymerisation 45,5 mol kg⁻¹s⁻¹

The examples I-II show that the acrylate polymerisation is a fast polymerisation which is less dependent on the functionality.

## Claims

1. UV or EB curable coating composition comprising a mono or multivalent carboxylic ester of a β-hydroxyalkylamide group containing compound and a photoinitiator, in which the carboxylic ester is derived from an α,β-ethylenically unsaturated carboxylic acid, **characterized in that** the compound is a compound according to formula (I): 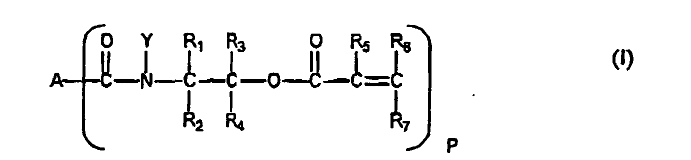 where:
A = a monovalent or polyvalent organic group which is derived from a saturated or an unsaturated (C₁-C₆₀) alkyl group, or derived from an (C₆-C₁₀) aryl group,
Y = hydrogen, a (C₁-C₆) alkyl group or 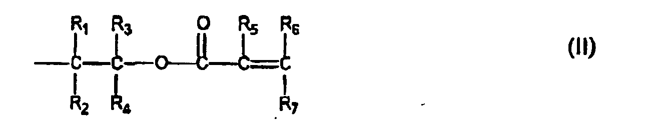
R₁, R₂, R₃, R₄ are identical or different, hydrogen or a linear, branched or cyclic (C₁₋C₈) alkyl chain,
R₅ = hydrogen, (C₁-C₈) alkyl, -CH₂OH or CH₂COOX,
R₈, R₇ = hydrogen, (C₁-C₈) alkyl, (C₆-C₁₀) aryl or COOX
X = hydrogen or (C₁-C₈) alkyl and
p = 1 or 2 and
wherein the photoinitiator has ketone functionality, the photoinitiator is an acyl phosphine or the photoinitiator is of the Norrish-II-type.

2. Composition according to Claim 1, **characterized in that** A is a monovalent organic group derived from a saturated (C₁-C₁₂) alkyl group or A is a polyvalent organic group derived from a saturated (C₂-C₁₀) alkyl group of a C₈-aryl group.

3. Composition according to any one of Claims 1-2, **characterized in that** Y is hydrogen or methyl, R₁, R₂, R₃, and R₄ are hydrogen or methyl, R₅ is hydrogen or (m)ethyl and R₆ and R₇ are hydrogen.

4. Composition according to any one of Claims 1-3, **characterized in that** the number average molecular weight of the compound is between 140 and 2500.

## Patentansprüche

1. UV- oder EB-härtbare Beschichtungszusammensetzung, welche einen ein- oder mehrwertigen Carbonsäureester einer β-Hydroxyalkylamidgruppenhaltigen Verbindung und einen Photoinitiator umfasst, in welcher der Carbonsäureester von einer α,β-ethylenisch ungesättigten Carbonsäure abgeleitet ist, **dadurch gekennzeichnet, dass** die Verbindung eine Verbindung gemäß der Formel (1) ist: 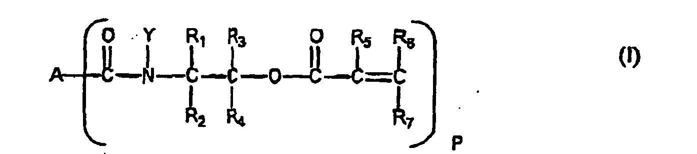
wobei:
A = eine einwertige oder mehrwertige organische Gruppe, welche von einer gesättigten oder ungesättigten (C₁-C₆₀)-Alkylgruppe abgeleitet ist, oder von einer (C₆-C₁₀)-Arylgruppe abgeleitet ist,
Y = Wasserstoff, eine (C₁-C₈)-Alkylgruppe oder 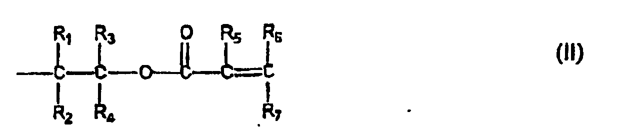
R₁, R₂, R₃, R₄ sind gleich oder verschieden, Wasserstoff oder eine geradkettige, verzweigtkettige oder cyclische (C₁-C₈)-Alkylkette,
R₅ = Wasserstoff, (C₁-C₅)-Alkyl, -CH₂OH oder CH₂COOX,
R₆, R₇ = Wasserstoff oder (C₁-C₈)-Alkyl, (C₆-C₁₀)-Aryl oder COOX,
X = Wasserstoff oder (C₁-C₈) Alkyl und
p = 1 oder 2 und
wobei der Photoinitiator eine Ketonfunktionalität aufweist, der Photoinitiator ein Acylphosphin ist oder der Photoinitiator vom Norrish-II-Typ ist.

2. Zusammensetzung nach Anspruch 1, **dadurch gekennzeichnet, dass** A eine einwertige organische Gruppe ist, welche von einer gesättigten (C₁-C₁₂)-Alkylgruppe abgeleitet ist, oder A eine mehrwertige organische Gruppe ist, welche von einer gesättigten (C₂-C₁₀)-Alkylgruppe einer C₆-Arylgruppe abgeleitet ist.

3. Zusammensetzung nach einem der Ansprüche 1 bis 2, **dadurch gekennzeichnet, dass** Y Wasserstoff oder Methyl ist, R₁, R₂, R₃ und R₄ Wasserstoff oder Methyl sind, R₅ Wasserstoff oder (M)ethyl ist und R₆ und R₇ Wasserstoff sind.

4. Zusammensetzung nach einem der Ansprüche 1 bis 3, **dadurch gekennzeichnet, dass** das zahlengemittelte Molekulargewicht der Verbindung zwischen 140 und 2500 liegt.

## Revendications

1. Composition de revêtement durcissable par ultraviolets ou par faisceau électronique, comprenant un ester carboxylique mono- ou multi-valent d'un composé contenant un groupe β-hydroxyalkylamide et un photoamorceur, dans laquelle l'ester carboxylique est dérivé d'un acide carboxylique à insaturation α,β-éthylénique, **caractérisée en ce que** le composé est un composé de formule (I) : 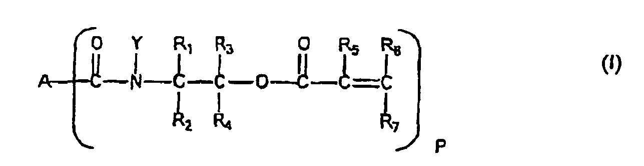
dans laquelle
A représente un groupe organique monovalent ou polyvalent qui est dérivé d'un groupe alkyle (en C₁ à C₈₀) saturé ou insaturé, ou dérivé d'un groupe aryle (en C₈ à C₁₀),
Y représente un atome d'hydrogène, un groupe alkyle en C₁ à C₈ ou 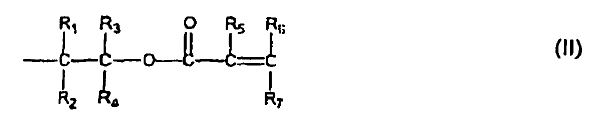
R₁, R₂, R₃, R₄ sont identiques ou différents et représentent un atome d'hydrogène, une chaîne alkyle (en C₁ à C₈) linéaire, ramifiée ou cyclique,
R₅ représente un atome d'hydrogène, un groupe alkyle (en C₁ à C₅), -CH₂OH ou -CH₂COOX,
R₆, R₇ représentent un atome d'hydrogène, un groupe alkyle (en C₁ à C8), un groupe aryle (en C₆ à C₁₀) ou -COOX,
X représente un atome d'hydrogène ou un groupe alkyle (en C₁ à C₈) et
p vaut 1 ou 2 et
dans laquelle le photoamorceur a une fonctionnalité cétone, le photoamorceur est une acylphosphine ou le photoamorceur est du type Norrish II.

2. Composition selon la, revendication 1, **caractérisée en ce que** A représente un groupe organique monovalent dérivé d'un groupe alkyle (en C₁ à C₁₂) saturé ou A représente un groupe organique polyvalent dérivé d'un groupe alkyle (en C₂ à C₁₀) saturé ou d'un groupe aryle en C₈.

3. Composition selon l'une quelconque des revendications 1 à 2, **caractérisée en ce que** Y représente un atome d'hydrogène ou un groupe méthyle, R₁, R₂, R₃ et R₄ représentent un atome d'hydrogène ou un groupe méthyle, R₅ représente un atome d'hydrogène ou un groupe (m)éthyle et R₆ et R₇ représentent un atome d'hydrogène.

4. Composition selon l'une quelconque des revendications 1 à 3, **caractérisée en ce que** la masse moléculaire moyenne en nombre du composé est comprise entre 140 et 2500.
